# EUROPÄISCHE PATENTSCHRIFT

(11) **EP 0 974 318 B1**
(45) Veröffentlichungstag und Bekanntmachung des Hinweises auf die Patenterteilung: **07.11.2007**
(21) Anmeldenummer: 99103862.1
(22) Anmeldetag: 01.03.1999
(51) Int. Cl.: A61F 2/38

(54) **Kniegelenkendoprothese**
Knee endoprosthesis
Endoprothèse de genou

(30) Priorität: 04.03.1998 DE 19809041
(43) Veröffentlichungstag der Anmeldung: 26.01.2000
(73) Patentinhaber: Engelbrecht, Eckart, Dr., 22359 Hamburg (DE); Hahn, Michael, Dipl.-Ing., 22417 Hamburg (DE)
(72) Erfinder: Engelbrecht, Eckart, Dr., 22359 Hamburg (DE); Hahn, Michael, Dipl.-Ing., 22417 Hamburg (DE)
(74) Vertreter: Heldt, Gert

(56) Entgegenhaltungen:
- EP-A- 0 178 445
- EP-A- 0 379 785
- EP-A- 0 582 514
- WO-A-96/24311
- DE-A- 2 545 821
- DE-A- 3 343 606
- DE-A- 4 119 226
- DE-U- 9 402 935
- FR-A- 2 662 931
- GB-A- 2 002 638
- US-A- 3 918 101
- US-A- 4 011 603
- US-A- 4 301 553
- US-A- 4 634 444
- US-A- 5 061 271
- US-A- 5 282 869

## Beschreibung

Die Erfindung betrifft eine Kniegelenkendoprothese aus einem Femurteil mit Gehäuse und einem Tibiateil mit einer an einem Ständer befestigten Achse, die in dem Gehäuse schwenkbar in einer Beugeebene gelagert ist, wobei der Femurteil mit einem Femurgleitlager auf einer mit dem Tibiateil verbundenen Tibiaplateau gleitend gelagert ist und an seinem dorsalen Ende eine steilere Krümmung als an seinem ventralen Ende aufweist und am Übergang von der steileren zur flacheren Krümmung eine Eindellung vorgesehen ist, in die in einer gestreckten Stellung eine Erhebung des Tibiaplateaus hineinragt.

Grundsätzlich lassen sich heute gekoppelte und ungekoppelte Kniegelenksendoprothesen unterscheiden. Die einzelnen Systeme haben ihre speziellen Indikationsbereiche. Beim Einbau von Kniegelenkendoprothesen bestehen nach wie vor Schwierigkeiten hinsichtlich einer exakten Positionierung bzw. der Koppelung der Komponenten. Einige Modelle erfordern außerdem eine relativ große Knochenresektion.

Bei eingekoppelten Kniegelenkendoprothesen werden das Femurteil und das Tibiateil durch eine separate Achse miteinander verbunden, wobei das Einführen der Achse eine zusätzliche Aufbohrung des Femurcondylus erfordert. Dies ist relativ umständlich und zeitaufwendig. Durch den zusätzlichen Knochenkanal wird zudem der verbleibende Knochenanteil geschwächt.

In der Patentanmeldeschrift GB 2 002 638 A wird eine Kniegelenkendoprothese beschrieben, deren Tibiateil einen Ständer mit zylindrischen Kopf besitzt der in einer entsprechenden Ausnehmung im Femurteil schwenkbar gelagert wird.

Die Ausnehmung im Femurteil wird seitlich durch eine Kappe und Schrauben verschlossen. Da in der Regel nach der Implantation der Prothese seitlich Knochen vorgelagert sind, kann die intraoperative Koppelung der Implantatteile nur durch eine Entfernung von Knochen erreicht werden. Eine einfach, knochenresektionsarme intraoperative Verbindung von Femur- und Tibiateil ist nicht möglich.

Durch die Anmeldeschrift DE 25 45 821 ist eine Kniegelenkendoprothese bekannt, die aus einem Oberteil, einem Mittelteil und einem Unterteil besteht. Ober- und Mittelteil werden über einen Ständer mit einer durch eine Bohrung am oberen Ende des Ständers hindurch geführten losen Achsen gekoppelt. Der Ständer ist fest mit dem Mittelteil verbunden und wird durch eine seitlich in die Bohrung einführbare Achse im Oberteil gehalten. Das Mittelteil wird mit dem Unterteil über einen Zapfen rotierbar verbunden. Durch den Aufbau der Prothese sind Schwenk- und Potationsbewegungen möglich. Auch bei dieser Prothese ist die intraoperative Koppelung der Komponenten schwierig und mit einer erhöhten Knochenresektion verbunden.

Aufgabe der vorliegenden Erfindung ist es daher, die bekannten Kniegelenkendoprothesen so zu verbessern, daß auf eine separa te Achse verzichtet werden kann und die Positionierung und Koppelung der Kniegelenkendoprothese vereinfacht bzw. verbessert und die erforderliche Knochenresektion minimiert wird. Die Erfindung soll insgesamt die Indikationsbreite des Systems erweitern.

Die Aufgabe wird erfindungsgemäß dadurch gelöst, dass der Ständer mit einer starren Achse in einen Zugangskanal des Gehäuses einsetzbar und im Gehäuse verriegelbar ist und das Femurgleitlager mindestens eine parallel zur Beugeebene verlaufende innere Auflagefläche aufweist, in deren Bereich die flachere Krümmung in den Bereich der steileren Krümmung verlagert wird.
Dadurch, daß die Achse fest an dem Ständer angeordnet ist und der Ständer mit der Achse zusammen in das Gehäuse eingesetzt werden kann, wird ein zusätzlicher Knochenkanal zum Einsetzen der Achse nicht benötigt. Die Verbindung von Femurteil und Tibiateil wird dadurch vereinfacht und die Operationszeit verringert. Durch die Verwendung eines Zugangskanals kann das achsseitige Ende des Ständers leicht in dem Lagerraum positioniert und eingerastet werden.

Gemäß einer weiteren bevorzugten Ausführungsform der Erfindung ist der Ständer in dem Gehäuse verriegelbar. Dabei ist der Zugangskanal mindestens teilweise von einer Abdeckung abdeckbar, die als Verriegelungsteil ausgebildet ist.

Durch die Verwendung einer den Innenraum schützenden Abdeckung als Verriegelungsteil, wird auf einfache und zuverlässige Weise der Ständer mit der Achse in dem Gehäuse verriegelt, so daß ein unbeabsichtigtes Lösen vermieden werden kann.

Nach einer weiteren bevorzugten Ausführungsform der Erfindung geht der Zugangskanal von einem Zugang an einer dem Tibiateil abgewandten Oberseite des Gehäuses aus und ist in einem der Patella abgewandten hinteren Bereich der Oberseite des Gehäuses angeordnet.

Dadurch ist es möglich, in starker Beugestellung das Tibiateil in das Femurteil einzusetzen und in den Normalstellungen bereits ohne Verriegelung eine formschlüssige sichere Verbindung zu erhalten.

Nach einer weiteren bevorzugten Ausführungsform der Erfindung ist der Zugangskanal mindestens teilweise von einer Abdeckung abdeckbar. Die Abdeckung ist dabei als Verriegelungsteil ausgebildet und in einer der Oberseite benachbarten Führung verschiebbar gelagert, wobei das Verriegelungsteil in Richtung einer quer zur Achse angeordneten Beugeebene verschiebbar ist.

Außer der Abdeckung ist ein in der Führung verschieblicher Deckel vorgesehen, der das Gehäuse vor dem unerwünschten Eindringen von Knochenzement beim Einzemtieren schützt. Dieser Deckel wird nach dem Zementieren und Erhärten des Zementes beseitigt und anschließend wird die Abdeckung in die Führung eingeschoben.

Gemäß einer weiteren bevorzugten Ausführungsform der Erfindung weist das Verriegelungsteil auf seiner dem Tibiateil zugewandten Unterseite eine auf die Achse abgestimmte Ausformung auf, die die Achse in Richtung ihrer Lagerstelle im Lagerraum beaufschlagt. Das Verriegelungsteil weist in einem Abstand zu der Ausformung eine parallel zur Achse angeordnete Längsnut auf, in die ein exzentrisch gelagerter Nocken eines Exzenters eingreift. Durch Verschwenken des Exzenters kann das verriegelungsteil leicht in seiner Führung verschoben werden. Durch die Ausformung an der Unterseite des Verriegelungsteiles rastet das Verriegelungsteil auf der Achse ein und ist somit gegen unbeabsichtigtes Entriegeln gesichert.

Das Femurgleitlager weist an den etwa parallel zur Beugeebene verlaufenden seitlichen Begrenzungen des Gehäuses Flügel auf, deren kufenförmige Auflageflächen sich entlang eines Bogens von einem dorsalen Ende in Richtung eines dem dorsalen Ende gegenüberliegenden ventralen Endes erstrecken. Der Bogen weist dabei in seinem dem dorsalen Ende zugewandten hinteren Bereich eine steile Krümmung auf, die in gestreckter Stellung etwa im Bereich einer Schaftlängsachse des Tibiateiles in eine flachere Krümmung übergeht. Der Übergang zwischen steiler und flacherer Krümmung ist als eine Eindellung ausgebildet, die in gestreckter Stellung an dem Tibiaplateau anschlägt. Das Tibiaplateau weist dabei eine Tibiaauflagefläche auf, die eine auf die Eindellung der Auflagefläche des Femurgleitlagers abgestimmte Erhebung aufweist, gegen die die Eindellung anschlägt. Die steiler gekrümmte Fläche ist dabei weiter in Richtung auf das Gehäuse geführt als bei den bisher bekannten Prothesen. Der äußere Bereich der kufenförmigen Auflageflächen wird damit im Bereich mit der steileren Krümmung über den Scheitelpunkt hinausgeführt, der bisher den Übergang zu dem Teil mit der flacheren Krümmung markierte. Die Eindellung, die innerhalb der kufenförmigen Auflagefläche entsteht, ist dadurch relativ flach ausgebildet, aber dennoch im Zusammenwirken mit der Erhebung als Anschlag geeignet, der die Streckstellung des Kniegelenkes bzw. der Kniegelenkendoprothese markiert. Je nach der Größe des Kniees kann dieser Punkt mehr oder minder weit in den stärker gekrümmten Teil verlagert werden. Durch die Eindellung einerseits und die von ihr beaufschlagte Erhebung andererseits wird die auf dem Tibiateil von einem Tibiaplateau aufgespannte Auflagefläche um etwa 50 % gegenüber einer nicht eingedellten Auflagefläche vergrößert und damit die bei der Übertragung von Kräften auftretende Flächenpressung erheblich herabgesetzt. Die Flügel gehen ventral zur Patella hin in einen kondylären Bereich über, der als Patellaschild ausgebildet ist, der eine flachere Krümmung als ein ihm gegenüber angeordneter dorsaler Bereich der Flügel aufweist. Von dem flacheren Bereich des Patellaschildes erstrecken sich in den Bereich der steileren Krümmung innere Auflageflächen, die die flachere Krümmung in den Bereich der steileren Krümmung verlagern.

Gemäß einer weiteren bevorzugten Ausführungsform der Erfindung gehen die Flügel ventral zur Patella hin in einen kondylären Bereich über, der als Patellaschild ausgebildet ist.

Das Patellaschild kann fest mit den kufenförmigen Auflageflächen der Flügel verbunden sein und mit den Flügeln eine Einheit bilden. Es kann jedoch auch losgelöst von den Flügeln oder modular ausgebildet sein. Schließlich ist es auch möglich, ganz auf das Patellaschild zu verzichten.

Gemäß einer weiteren bevorzugten Ausführungsform der Erfindung ist dem Femurgleitlager ventral in einem der Patella zugewandten kondylären Bereich eine Patellainterpositionsprothese vorgelagert. Die Patellainterpositionsprothese ist dabei in einem ventralen Bereich des Tibiaplateaus angelenkt und über mindestens ein Zwischenstück mit dem Tibiaplateau verbunden.

Durch die zwischen kondylärem Femurgleitlager und Patellarückscite angeordnete Patellainterpositionsprothese wird vorteilhaft erreicht, daß der eigentliche Gleitvorgang zwischen Patellarückfläche und Femurgleitlager auf den Bereich der dem kondylären Prothesengleitlager zugewandten Rückfläche der Patellainterpositionsprothese verlagert wird. Zwischen der Patellarückseite und der Patellainterpositionsprothese kommt es dann vergleichsweise zu nur kleinen Relativbewegungen. Auf einen Patellarückflächenersatz bzw. auf eine Beschichtung der Patellarückfläche mit beispielsweise Polyäthylen kann dann verzichtet werden. Die Anlenkung der Patellainterpositionsprothese über ein Zwischenstück ermöglicht eine Art Doppelgelenk, das eine günstigere Anlage der Patellainterpositionsprothese gegen das Femurgleitlager ermöglicht.

Nach einer weiteren bevorzugten Ausführungsform der Erfindung weisen die Flügel auf ihrer der Auflagefläche abgewandten Innenfläche als äußere Begrenzung eine von der Innenfläche weggerichtete Randlippe auf.

Die nach innen gerichteten Randlippen bilden damit kleine Vorsprünge, zwischen denen der Zement gefangen wird, wenn das Femurteil montiert wird. In diesem Fall bildet sich ein gepreßtes Bett aus Knochenzement auf dem Innenteil der Kufe aus.

Gemäß einer weiteren bevorzugten Ausführungsform der Erfindung weisen die Innenflächen eine Vielzahl von Ausformungen zur besseren Verbindung mit dem an ihnen anliegenden Material auf, die als gewellte Schneidkanten ausgebildet sind. Dadurch ist es auch möglich, bei zementfreien Prothesen eine bessere Verkrallung der Knochenmasse mit dem Implantat herbeizuführen.

Nach einer weiteren bevorzugten Ausführungsform der Erfindung weisen das Femurteil und das Tibiateil an ihren einander abgewandten Enden einen auswechselbaren Schaft auf. Die Schäfte sind mit ihrem gelenkseitigen Ende auf einen fest mit dem Femur- bzw. Tibiateil verbundenen Kegel aufsteckbar. Die Kegel sind als stumpfe Kegel ausgebildet und die Schäfte weisen eine Längsbohrung auf,durch die eine Schraube einführbar ist, mit der die Schäfte mit dem jeweiligen Konus verschraubbar sind.

Durch die auswechselbaren Schäfte kann bei relativ geringer Lagerhaltung die Kniegelenkendoprothese an unterschiedlich große Knochen angepaßt werden.

Gemäß einer weiteren bevorzugten Ausführungsform der Erfindung ist den Schäften jeweils ein in den Knochenkanal einsetzbares Führungssystem vorlagerbar. Das Führungssystem weist eine von einer zentralen Bohrung durchzogene Führungsspindel auf, an deren dem Schaft zugewandtem Ende ein den Knochenkanal gegen Knochenzement abdichtender Teller angeordnet ist. Die Führungsspindel weist an ihrem dem Teller abgewandten Ende beispielsweise ein sich am Knochenkanal seitlich abstützendes Rossettensystem auf. Die Führungsspindel ist mit einem Einführinstrument in den Knochenkanal einführbar. Nach dem Entfernen des Einführinstrumentes ist ein Führungsstab mit einem vorderen Ende in die zentrale Bohrung der Führungsspindel einsetzbar. Der Führungsstab weist ein zu dem vorderen Ende abgewandtes hinteres Ende auf, über das der Schaft in den Knochenkanal einsetzbar ist. Die Ausbildung der Spindel ist so gewählt, daß sie sich optimal der Krümmung des Markraumes anpaßt.

Durch das Führungssystem ist es möglich, den tieferen Bereich des Knochenkanales frei von Verankerungsmasse, sei es Knochenzement oder Knochengewebe zu halten und zugleich eine gute Führung des Schaftes zu erzielen. Der den Knochenkanal abdichtende Teller verschließt den Knochenkanal so, daß sich an dem Teller kein Knochenzement vorbeidrücken kann. Bei einem eventuell notwendigen späteren Austausch der Kniegelenkendoprothese ist der hintere Teil des Knochenkanales frei von Knochenzement und das Femur- bzw. Tibiateil kann leichter entfernt werden. Die Gefahr der Schädigung des Knochenkanales ist dabei geringer und es ist leichter möglich, erneut eine Kniegegelenkendoprothese einzusetzen.

Weitere Einzelheiten der Erfindung ergeben sich aus der nachfolgenden ausführlichen Beschreibung und den beigefügten Zeichnungen, in denen bevorzugte Ausführungsformen der Erfindung beispielsweise veranschaulicht sind.

In den Zeichnungen zeigen:

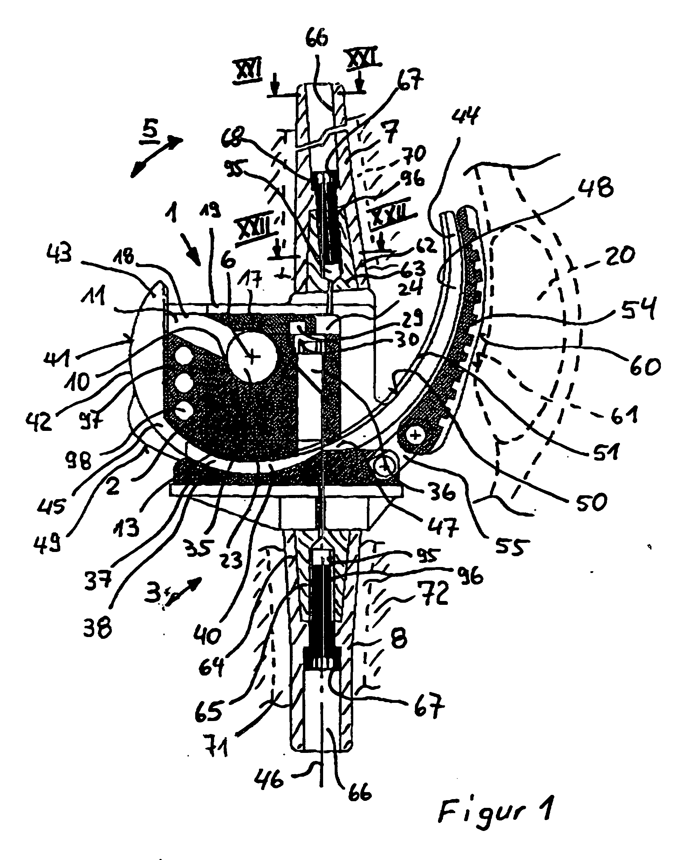

- Figur 1:: Eine Seitenansicht einer Kniegelenkendoprothese mit schraffiert dargestellten Kunststoffteilen und mit gestrichelt angedeuteter Patella im Schnitt,

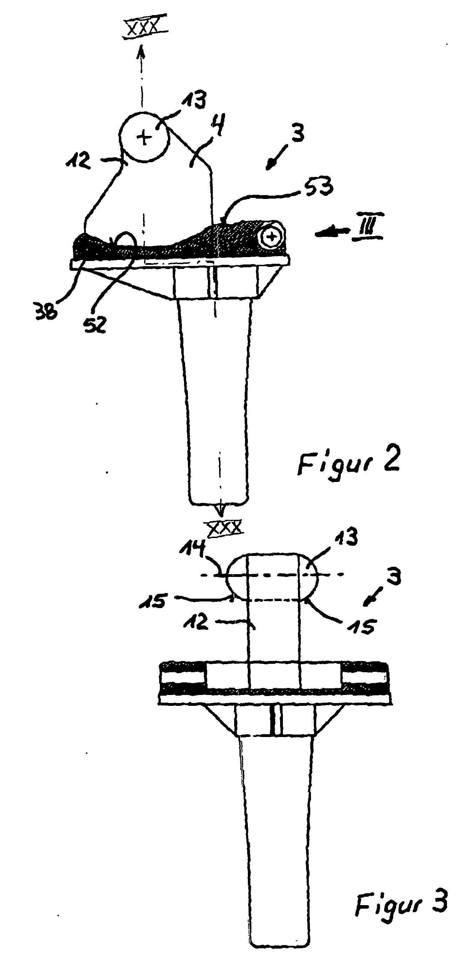

- Figur 2:: eine Seitenansicht eines Tibiateiles von Figur 1,
- Figur 3:: eine Vorderansicht des Tibiateiles von Figur 2 aus Richtung III,

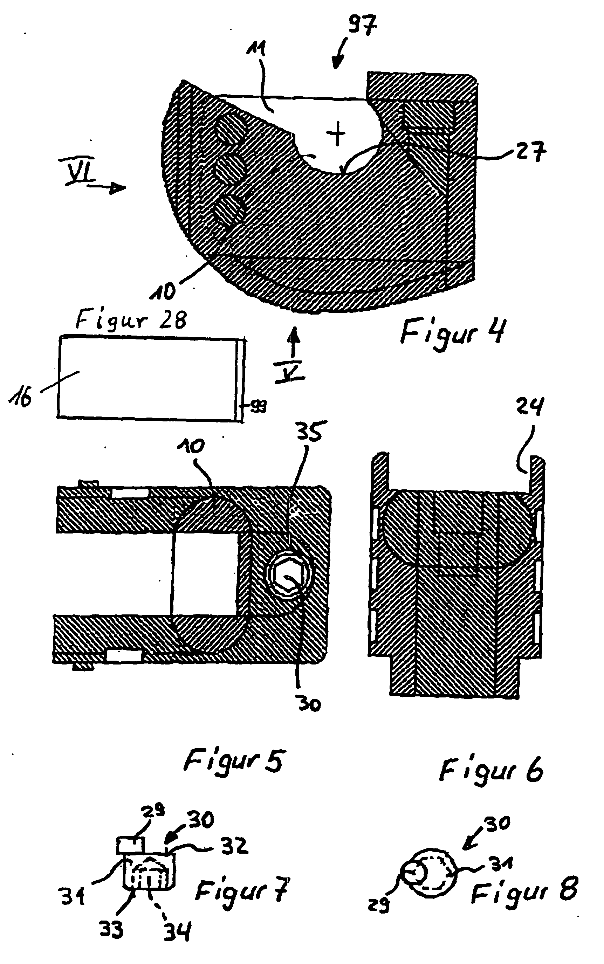

- Figur 4:: eine Seitenansicht eines Kunststoffeinsatzes von Figur 1 in vergrößerter Darstellung,
- Figur 5:: eine Untersicht unter den Kunststoffeinsatz von Figur 4 aus Richtung V in vergrößerter Darstellung,
- Figur 6:: eine Rückansicht des Kunststoffeinsatzes von Figur 4 aus Richtung VI in vergrößerter Darstellung,
- Figur 7:: eine Seitenansicht eines Exzenters von Figur 1 in vergrößerter Darstellung,
- Figur 8:: eine Draufsicht auf den Exzenter von Figur 7,

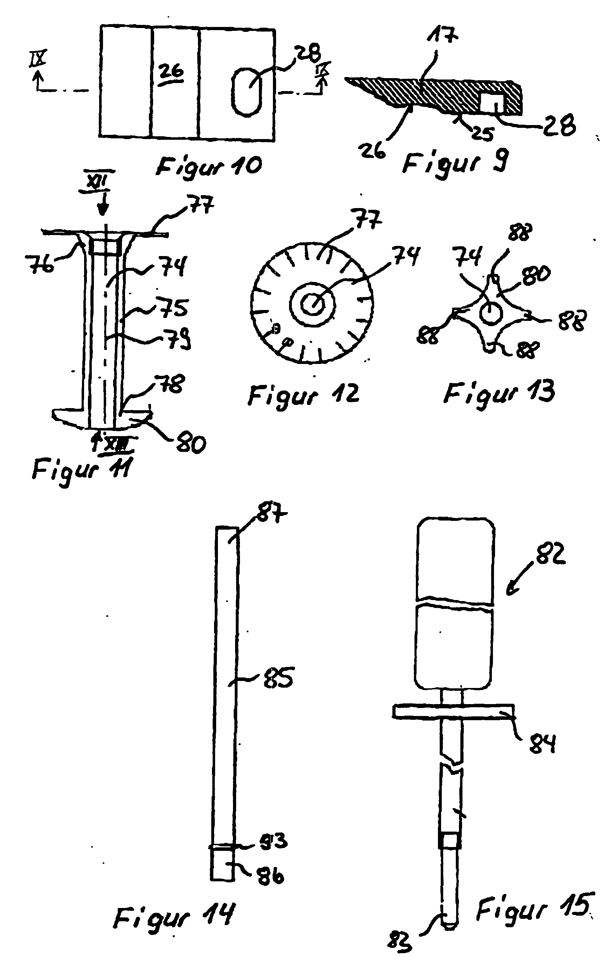

- Figur 9:: ein Längsschnitt durch ein Verriegelungsteil von Figur 10 entsprechend der Schnittlinie IX-IX in Figur 10,
- Figur 10:: eine Untersicht unter das Verriegelungsteil von Figur 9,
- Figur 11:: eine Seitenansicht einer Zentrierspindel in vergrößerter Darstellung im Schnitt,
- Figur 12:: eine Draufsicht auf die zentrierspindel von Figur 11 aus Richtung XII,
- Figur 13:: eine Draufsicht auf die Zentrierspindel von Figur 11 aus Richtung XIII,
- Figur 14:: eine Seitenansicht eines Führungsstabes,
- Figur 15:: eine Seitenansicht eines Einführinstrumentes,

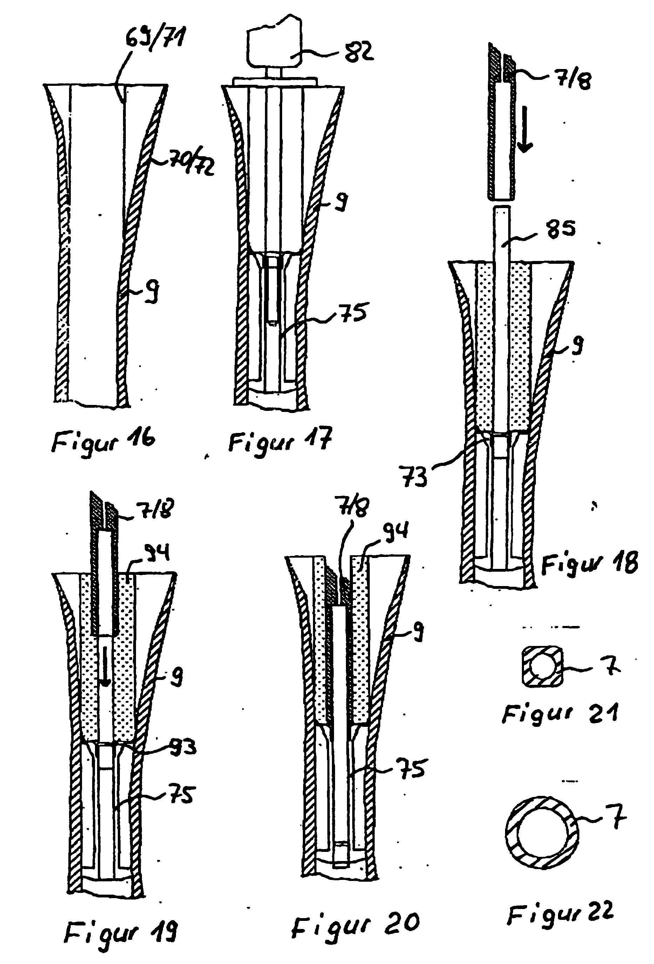

- Figur 16:: eine Schnittdarstellung eines aufgebohrten Röhren-knochens,
- Figur 17:: eine Schnittdarstellung des Röhrenknochens von Figur 16 mit eingesetzter Zentrierspindel und aufgesetztem Einführinstrument,
- Figur 18:: eine Schnittdarstellung des Röhrenknochens von Figur 16 mit eingesetzter Zentrierspindel, Führungsstab, eingebrachtem Knochenzement und Schnittdarstellung eines Prothesenschaftes im Ausriß,
- Figur 19:: eine Schnittdarstellung des Röhrenknochens von Figur 18 mit auf die Führungsstange aufgesetztem Schaft,
- Figur 20:: eine Schnittdarstellung des Röhrenknochens von Figur 19 mit Schaftdarstellung in Endposition,
- Figur 21:: einen Querschnitt des Schaftes von Figur 1 entlang der Linie XXI - XXI geschnitten,
- Figur 22:: einen Querschnitt des Schaftes von Figur 1 entlang der Linie XXII - XXII geschnitten,
- Figur 23:: eine Seitenansicht eines Tibiateiles,

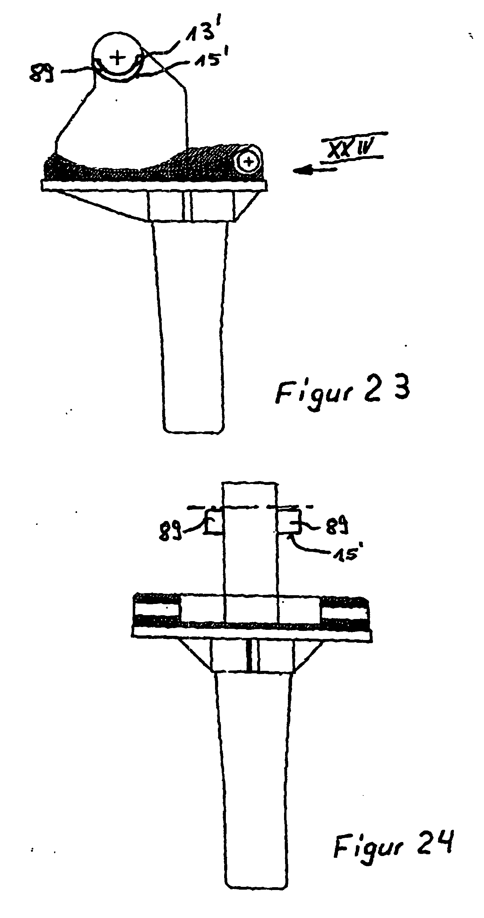

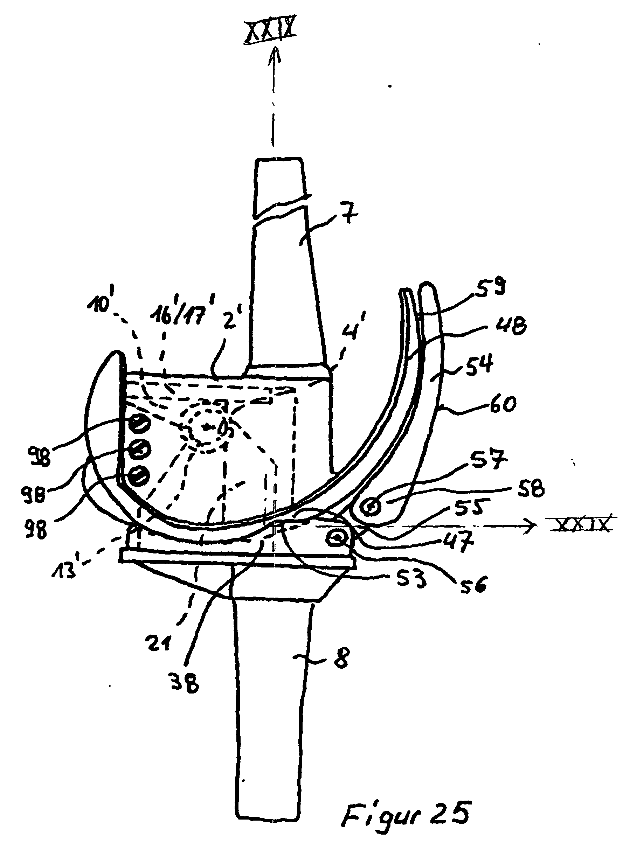

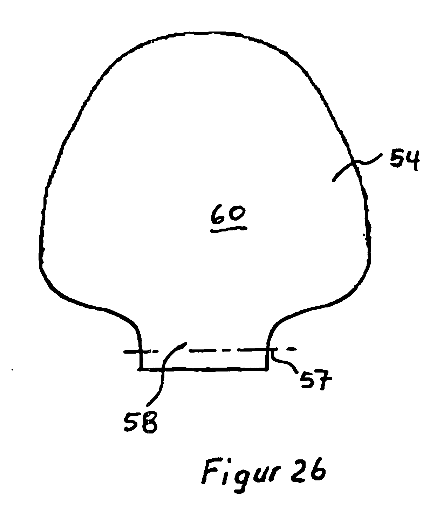

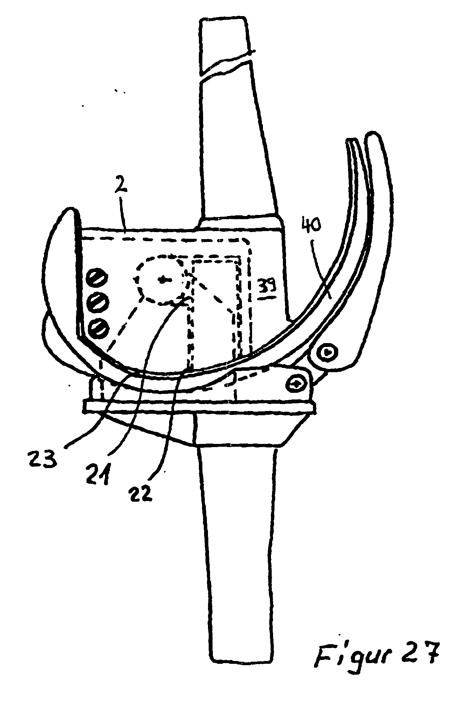

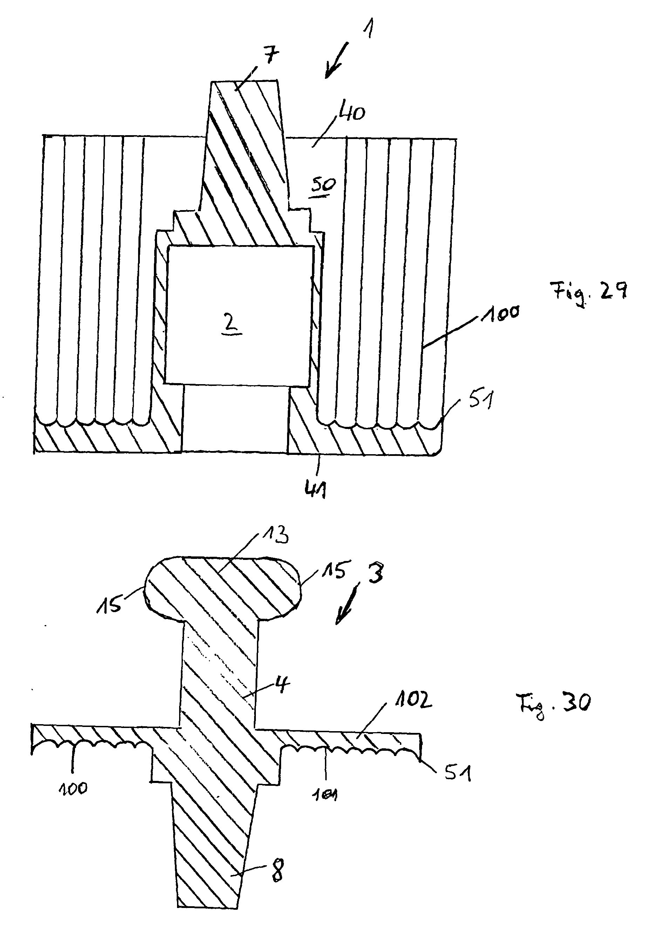

- Figur 24:: eine Vorderansicht des Tibiateiles von Figur 23 aus Richtung XXIV,
- Figur 25:: eine Seitenansicht einer Kniegelenkendoprothese mit einer gestrichelt angedeuteten Achse mit Kreisringsegmenten,
- Figur 26:: eine Vorderansicht einer Interpositionsprothese,
- Figur 27:: eine Seitenansicht einer Kniegelenkendoprothese mit gestrichelt angedeutetem Zugangskanal von einer Unterseite her,
- Figur 28:: eine Draufsicht auf eine Abdeckung, Figur 29: ein Längsschnitt durch einen Femurteil entlang der Schnittlinie XXIX-XXIX in Figur 25 und
- Figur 30:: ein Längsschnitt durch einen Tibiateil entlang der Schnittlinie XXX-XXX in Figur 2.

Eine Kniegelenkendoprothese besteht im wesentlichen aus einem Femurteil (1) mit einem Gehäuse (2) und aus einem Tibiateil (3) mit einem Ständer (4).

Das Femurteil (1) und das Tibiateil (3) sind in einer Beugeebene (5) um eine quer zur Beugeebene (5) angeordnete Kniegelenk-Schwenkachse (6) gegeneinander zwischen einer gestreckten und einer gebeugten Stellung verschwenkbar.

Das Femurteil (1) weist einen Femurschaft (7) und das Tibiateil (3) weist einen Tibiaschaft (8) auf. Die beiden Schäfte (7, 8) weisen in einander abgewandte Richtungen und dienen zur Befestigung der Teile (1, 3) in einem ihnen jeweils benachbarten Knochen (9, 70, 72).

Das Gehäuse (2) weist einen Lagerraum (10) mit einem Zugangskanal (11) für ein achsseitiges Ende (12) des Ständers (4) auf. Der Lagerraum (10) ist als ein Kunststoffeinsatz (97) ausgebildet, der mit Gehäuseschrauben (98) am Gehäuse befestigt ist. An dem achsseitigen Ende (12) des Ständers (4) ist eine Achse (13) fest angeordnet, deren Längsachse (14) im montierten Zustand mit der Kniegelenk-Schwenkachse (6) zusammenfällt bzw. diese bildet.

Die Achse (13) weist an ihren einander abgewandten Enden Führungsflächen (15) auf, die im wesentlichen kugelabschnittsförmig ausgebildet sind.

Der Ständer (4) ist mit der Achse (13) formschlüssig in das Gehäuse (2) einsetzbar. Dabei rastet die Achse (13) mit ihren Führungsflächen (15) in entsprechende Ausformungen des Lagerraumes (10) ein. Der Zugangskanal (11) ist von einer Abdeckung (16) abdeckbar, die vorübergehend in eine Führung (24) eingeschoben wird, um während der Einzementierung des Femurteils (1) ein Eindringen von Knochenzement in das Gehäuse (2) zu verhindern. Die Abdeckung (16) besitzt eine leichte Schwelle (99), die einerseits in eingelegten Zustand der Abdeckung (16) den Femurschaft (7) gegen Knochenzement schützt und andererseits eine Handhabe zum Verschieben der Abdeckung (16) bildet. Der Zugangskanal (11) geht von einem Zugang (18) an einer dem Tibiateil (3) abgewandten Oberseite (19) des Gehäuses (2) aus.

Der Zugang (18) ist in einem der Patella (20) abgewandten hinteren Bereich der Oberseite (19) des Gehäuses angeordnet.

Grundsätzlich ist es aber auch möglich, den Zugangskanal (21) von einem Zugang (22) an einer dem Tibiateil (3) zugewandten Unterseite (23) des Gehäuses (2) ausgehen zu lassen. Dabei ist es zweckmäßig, den Zugang (22) in einem der Patella (20) zugewandten vorderen Bereich der Unterseite (23) des Gehäuses (2) anzuordnen.

Nachdem der Knochenzement sich verfestigt hat, wird die Abdeckung (16) vom Zugangskanal (17) abgezogen und durch ein Verriegelungsteil (17) ersetzt. Dieses ist in der Oberseite (19) benachbarten Führung (24) verschiebbar gelagert. Sowohl das Verriegelungsteil (17) als auch die Abdeckung (16) sind in Richtung der Beugeebene (5) verschiebbar. Das Verriegelungsteil (17) weist auf seiner dem Tibiateil (3) zugewandten Unterseite (25) eine auf die Achse (13) abgestimmte Ausformung (26) auf, die die Achse (13) in Richtung ihrer Lagerstelle (27) im Lagerraum (10) beaufschlagt. Das Verriegelungsteil (17) weist in einem Abstand zu der Ausformung (26) eine etwa parallel zur Achse (13) angeordnete Längsnut (28) auf, in die ein exzentrisch gelagerter Nocken (29) eines Exzenters (30) eingreift. Der Exzenter (30) besteht im wesentlichen aus einer zylindrischen Scheibe (31) mit einer der Oberseite (19) zugewandten oberen Seite (32) auf der der Nocken (29) exzentrisch angeordnet ist. Die zylindrische Scheibe weist an ihrer der oberen Seite (32) abgewandten unteren Seite (33) eine Aufnahme (34) für ein Werkzeug auf, die beispielsweise als Innensechskant ausgebildet sein kann. Der Exzenter (30) ist in einer Exzenteraufnahme (35), die an einem oberen Ende eines von einer der Oberseite (19) des Gehäuses (2) abgewandten Unterseite (23) des Gehäuses (2) in Richtung Oberseite (19) ausgehenden Kanales (36) angeordnet ist, verschwenkbar gelagert. Durch Verschwenken des Exzenters (30) mit einem in den Kanal (36) einführbaren Werkzeug läßt sich das Verriegelungsteil (17) in seiner Führung (24) verschieben. Das Femurteil (1) weist ein Femurgleitlager (37) und das Tibiateil (3) weist ein Tibiaplateau (38) als Gegenlager auf. Das Femurteil (1) weist an seitlichen Begrenzungen (39) des Gehäuses (2) Flügel (40) auf, deren kufenförmige Auflageflächen (41) sich entlang eines Bogens (42) von einem dorsalen Ende (43) in Richtung eines dem dorsalen Ende (43) gegenüberliegenden ventralen Endes (44) erstrecken. Der Bogen (42) weist in seinem dem dorsalen Ende (43) zugewandten hinteren Bereich (45) eine steile Krümmung auf, die in gestreckter Stellung etwa im Bereich einer Schaftlängsachse (46) des Tibiateiles (3) bzw. des Tibiaschaftes (8) in eine flachere Krümmung übergeht. Der Übergang zwischen steiler und flacherer Krümmung ist als eine Eindellung (47) ausgebildet, die in gestreckter Stellung an dem Tibiaplateau (38) anschlägt.

Die Flügel (40) gehen ventral in einen kondilären Bereich über, der als Patellaschild (48) ausgebildet ist. Das Femurgleitlager (37) weist neben den Auflageflächen (41) der Flügel (40) zwei in einem Abstand zueinander verlaufende innere Auflageflächen (49) auf. Die inneren Auflageflächen (49) verlagern die flachere Krümmung in den Bereich der steilen Krümmung.

Die Flügel (40) weisen auf ihrer der Auflagefläche (41) abgewandten Innenfläche (50) als äußere Begrenzung eine von der Innenfläche (50) weggerichtete Randlippe (51) auf, die als umlaufender Rand ausgebildet ist.

Die von der Randlippe (51) umgrenzte Innenfläche (50) weist eine Vielzahl von Ausformungen zur besseren Verbindung mit dem an ihr anliegenden Material auf. Die Ausformungen können beispielsweise als gewellte Schneidkanten (100) ausgebildet sein.

In ähnlicher Weise besitzt der Tibiateil (3) auf einer der Tibia (72) zugewandten Unterfläche (101) ihrer sich auf der Tibia (72) abstützenden Abstützfläche (102) Ausformungen, die eine Verbindung der Unterfläche (101) mit einer entsprechend präparierten Abschlußfläche der Tibia (72) begünstigen. Auch diese Ausformungen sind zweckmäßigerweise als Schneidkanten (100) ausgebildet, die sich in die Abschlußfläche der Tibia (72) einschneiden. Außerdem besitzt auch die Unterfläche (101) an ihren äußeren Begrenzungen eine Randlippe (51), die als umlaufender Rand ausgebildet ist. Diese Randlippe (51) sorgt dafür, daß der zwischen der Auflagefläche der Tibia (72) und der Unterfläche (101) eingebrachte Knochenzement nicht über einen die Abstützfläche begrenzenden Rand hinaustritt.

Das Tibiaplateau (38) weist eine Tibiaauflagefläche (52) auf, die auf das Femurgleitlager (37) abgestimmt ist. Die Tibiaauflagefläche (52) weist eine auf die Eindellung (47) der Auflagefläche (41) des Femurgleitlagers (37) abgestimmte Erhebung (53) auf, die der Eindellung (47) als Anschlag dient.

Dem Femurgleitlager (37) ist ventral in einem der Patella zugewandten kondylären Bereich zwischen Patellaschild (48) und Patella (20) eine Patellainterpositionsprothese (54) vorgelagert. Die Patellainterpositionsprothese (54) ist in einem ventralen Bereich des Tibiaplateaus (38) angelenkt. Die Patellainterpositionsprothese (54) ist über ein Zwischenstück (55) mit dem Tibiaplateau (38) verbunden. Das Zwischenstück (55) ist um eine erste Schwenkachse (56), die parallel zu der Kniegelenkschwenkachse (6) verläuft, schwenkbar mit dem Tibiaplateau (38) verbunden. An dem dem Tibiaplateau (38) abgewandten Ende ist das Zwischenstück (55) um eine parallel zur ersten Schwenkachse (56) verlaufende zweite Schwenkachse (57) schwenkbar mit einem unteren Ende (58) der Patellainterpositionsprothese (54) verbunden. Die Patellainterpositionsprothese (54) liegt mit einer der Patella (20) abgewandten Rückfläche (59) gleitbar auf dem Patellaschild (48) an. Auf ihrer der Rückfläche (59) abgewandten Seite weist die Patellainterpositionsprothese (54) eine Auflagefläche (60) auf, auf die die Patella (20) mit ihrer der Patellainterpositionsprothese (54) zugewandten Patellarückfläche (61) großflächig lagerbar ist.

Der Femurschaft (7) ist mit seinem gelenkseitigen Ende (62) auf einen fest mit dem Femurteil (1) verbundenen Kegel (63) aufsteckbar. Der Tibiaschaft (8) ist mit seinem gelenkseitigen Ende (64) auf einen fest mit dem Tibiateil (3) verbundenen zweiten Kegel (65) aufsteckbar. Die Kegel (63, 65) sind als stumpfe Kegel ausgebildet, die eine zentrale Bohrung (95) und ein Innengewinde (96) aufweisen. Die Schäfte (7, 8) weisen eine Längsbohrung (66) auf, durch die eine Schraube (67) einführbar ist, mit der die Schäfte (7, 8) mit dem jeweiligen Kegel (63, 65) verschraubbar sind.

Die Schraube (67) weist in ihrer Längsrichtung eine Entlüftungsbohrung (68) auf. Das Femurteil (1) ist mit seinem Femurschaft (7) in einem vorbereiteten Knochenkanal (69) des benachbarten Femurs (70) einzementierbar. Das Tibiateil (3) ist mit seinem Tibiaschaft (8) in einem vorbereiteten Knochenkanal (71) der benachbarten Tibia (72) einzementierbar.

Den Schäften (7, 8) ist ein in den Knochenkanal (69, 71) einsetzbares Führungssystem (73) vorgelagert. Das Führungssystem (73) weist eine von einer zentralen Bohrung (74) durchzogene Führungsspindel (75) auf. Die Führungsspindel (75) weist an einem dem Schaft (7, 8) zugewandten ersten Ende (76) einen den Knochenkanal (71) gegen Knochenzement abdichtenden Teller (77) auf. Der Teller (77) ist an seinem Rand flexibel ausgebildet, damit er sich beim Einsetzen an die Wandungen des ihn umgebenden Knochenkanals (69, 71) anpassen kann. Der Teller dient zugleich der Führung der Führungsspindel (75) im Knochenkanal (69, 71).

An ihrem dem ersten Ende (76) abgewandten zweiten Ende (78) weist die Führungsspindel (75) ein sich am Knochenkanal (69, 71) abstützendes Rossettensystem (80) auf. Das Rossettensystem (80) ist quer zu einer Längsachse (79) sternförmig ausgebildet und weist beispielsweise vier um etwa 90° zueinander versetzte Auflageecken (88) auf. Zwischen den vier Auflageecken (88) bestehen relativ große Räume, durch die die Gefäße verlaufen können, die der Knochenernährung dienen. Diese Gefäße können dann bis zum anderen Ende der Führungsspindel (75) wachsen.

Das Rossettensystem (80) dient ebenfalls der Führung der Führungsspindel (75) im Knochenkanal (69, 71).

Die Führungsspindel (75) ist mit einem Einführinstrument (82) in den Knochenkanal (69, 71) einführbar. Die Führungsspindel (75) ist auf ein vorderes Ende (83) des Einführinstrumentes (82) aufschraubbar. Das Einführinstrument (82) weist einen Anschlag (84) auf, der auf die Tiefe des in den Knochenkanal (69, 71) einzuführenden Schaftes (7, 8) einstellbar ist.

Ein Führungsstab (85) ist mit einem vorderen Ende (86) in die zentrale Bohrung (74) der Führungsspindel (75) einsetzbar. Über ein dem vorderen Ende (86) abgewandtes hinteres Ende (87) ist der Schaft (7, 8) in den Knochenkanal (69, 71) einsetzbar.

Nach einer anderen Ausführungsform der Erfindung weist die Achse (13') an ihren einander abgewandten Enden Führungsflächen (15') auf, die als Kreisringabschnitte (89) ausgebildet sind. Die Achse (13') ist somit nur noch hinsichtlich ihrer äußeren Begrenzung auf den Ständer (4') des Tibiateiles (3) ausgebildet. Sie bildet die Führungsflächen (15') auf einen Radius, der von dem Drehkreis des Femurteiles (1) gegenüber dem Tibiateil (3) vorgegeben ist. Die Kreisringabschnitte (89) werden in einer entsprechenden Gegenform des Lagerraumes (10') des Gehäuses (2') geführt. Eine Verblockung bzw. Verriegelung gegen unbeabsichtigtes Lösen erfolgt durch eine als Verriegelungsteil (17') ausgebildete Abdeckung (16'). Um zu verhindern, daß in der Zementierphase Knochenzement in das Gehäuse (2') eindringt, wird das Gehäuse (2') während der Zementierphase nach oben durch die provisorische Abdeckung (16) abgeschlossen, die anstelle des Verriegelungsteiles (17') in das Gehäuse (2') eingesetzt wird. Diese provisiorische Abdeckung (16') sorgt dafür, daß der Zement von dem Innenbereich des Gehäuses (2') abgehalten wird. Zu diesem Zweck besitzt er auf seiner dem Gehäuse (2') abgewandten Oberseite die Schwelle (99) als Schiebevorsprung, die den Knochenzement aus dem Bereich der oberen Öffnung des Gehäuses (2') verdrängt. Nach Beendigung wird diese provisiorische Abdeckung (16') entfernt und durch das Verriegelungsteil (17') ersetzt, das das Gehäuse (2') endgültig abschließt und auch nach oben stabilisiert.

Zum Einsetzen der Teile (1, 3) in den sie umgebenden Knochen (9, 70, 72) werden die Knochen für die Aufnahme der Schäfte (7, 8) vorbereitet und die notwendigen Knochenkanäle (69, 71) hergestellt. Auf die Führungsspindel (75) wird das Einführinstrument (82) aufgeschraubt und die Führungsspindel (75) in den Knochenkanal (69, 71) eingeführt. Dabei kann das Einführinstrument (82) auf die Tiefe des jeweils gewählten Schaftes (7, 8) durch einen verschieblichen Anschlag (84) eingestellt werden. Nach dem Einsetzen der Führungsspindel (75) wird das Einführinstrument (82) von der Führungsspindel (75) abgeschraubt. Sodann wird in die Führungsspindel (75) der Führungsstab (85) eingeführt, beispielsweise mit Hilfe eines Bajonett-Verschlusses (93). Dadurch ist gewährleistet, daß der anschließend auf den Führungsstab (85) aufgeschobene Schaft (7, 8) der Kniegelenkendoprothese genau auf dem Führungsstab (85) geführt wird und zwar entsprechend der Richtung, die von der Führungsspindel (75) vorgegeben ist und damit im Knochen (9) bzw. im Knochenkanal (69, 71) zentriert wird. Nunmehr wird der Knochenzement (94) in den Knochenkanal (69, 71) eingeführt. Dabei ist darauf zu achten, daß in dem Knochenzement (94) möglichst wenig Luft gelöst ist. Nach dem Einführen des Knochenzementes (44) wird der durchbohrte Schaft (7, 8) der Kniegelenkendoprothese auf dem Führungsstab (85) aufgeschoben. Dabei wird darauf geachtet, daß sich innerhalb des durchbohrten Schaftes (7, 8) keine Luft komprimieren kann, die gegebenenfalls in den noch feuchten Knochenzement (94) eindringen kann. Vielmehr ist dafür gesorgt, daß durch die Längsbohrung (66) und durch die Entlüftungsbohrung (68) der Schraube (67) die Luft beim Einschieben des Schaftes (7, 8) auf den Führungsstab (85) austreten kann und sich nicht im Knochenzement (94) verteilen kann. Die Luft tritt somit beim Femurteil (1) über das Gehäuse (2, 2') aus. Beim Tibiateil (3) tritt die Luft im Bereich des Tibiaplateaus (38) aus.

Nach dem Einzementieren werden Tibiateil (3) und Femurteil (1) in eine starke Beugestellung gebracht und der Ständer (4, 4') wird in das Gehäuse (2, 2') eingesetzt, gegebenenfalls eingerastet und anschließend mit dem Verriegelungsteil (17, 17') verriegelt.

## Patentansprüche

1. Knieendoprothese aus einem Femurteil (1) mit Gehäuse (2) und einem Tibiateil (3) mit einer an einem Ständer (4) befestigten Achse (13), die in dem Gehäuse 2 schwenkbar in einer Beugeebene (5) gelagert ist, wobei der Femurteil (1) mit einem Femurgleitlager (37) auf einem mit dem Tibiateil (3) verbundenen Tibiaplateau gleitend gelagert ist und an seinem dorsalen Ende (43) eine steilere Krümmung als an seinem ventralen Ende (44) aufweist und am Übergang von der steileren zu der flacheren Krümmung eine Eindellung (47) vorgesehen ist, in die in einer gestreckten Stellung eine Erhebung (53) des Tibiaplateaus (38) hineinragt **dadurch gekennzeichnet, dass** der ständer (4) mit einer fest an dem Ständer eingeordneten Achse (13) in einen Zugangskanal (11, 21) des Gehäuses (2) ein setzbar und im Gehäuse (2) verriegelbar ist und das Femurgleitlager (37) mindestens eine parallel zur Beugeebene (5) verlaufende innere Auflagefläche (49) aufweist, in deren Bereich die flachere Krümmung in den Bereich der steileren Krümmung verlagert wird.

2. Kniegelenkendoprothese nach Anspruch 1, **dadurch gekennzeichnet, dass** das Gehäuse (2, 2') einen Lagerraum aufweist, in den die Achse (13) einrastbar einsetzbar ist.

3. Kniegelenkendoprothese nach einem der Ansprüche 1 oder 2, **dadurch gekennzeichnet, daß** der Ständer (4, 4') in dem Gehäuse (2, 2', 2'') verriegelbar ist.

4. Kniegelenkendoprothese nach einem der Ansprüche 1 bis 3, **dadurch gekennzeichnet, daß** der Zugangskanal (11, 21) mindestens teilweise von einer Abdeckung (16, 16') abdeckbar ist.

5. Kniegelenkendoprothese nach Anspruch 4, **dadurch gekennzeichnet, daß** die Abdeckung (16, 16') als Verriegelungs teil ausgebildet ist.

6. Kniegelenkendoprothese nach einem der Ansprüche 1 bis 5, **dadurch gekennzeichnet, daß** der Zugangskanal (11'') von einem Zugang (22) an einer dem Tibiateil (3) zugewandten Unterseite (23) des Gehäuses (2'') ausgeht.

7. Kniegelenkendoprothese nach Anspruch 6, **dadurch gekennzeichnet, daß** der Zugang (22) in einem der Patella (20) zugewandten vorderen Bereich der Unterseite (23) des Gehäuses (2'') angeordnet ist.

8. Kniegelenkendoprothese nach einem der Ansprüche 5 bis 7, **dadurch gekennzeichnet, daß** das Verriegelungsteil (17, 17') in den Zugangskanal (11, 21) einsetzbar ist.

9. Kniegelenkendoprothese nach einem der Ansprüche 1 bis 8, **dadurch gekennzeichnet, daß** der Zugangskanal (11) von einem Zugang (18) an einer dem Tibiateil (3) abgewandten Oberseite (19) des Gehäuses (2, 2') ausgeht.

10. Kniegelenkendoprothese nach Anspruch 9, **dadurch gekennzeichnet, daß** der Zugang (18) in einem der Patella (20) abgewandten hinteren Bereich der Oberseite (19) des Gehäuses (2, 2') angeordnet ist.

11. Kniegelenkendoprothese nach Anspruch 9 oder 10, **dadurch gekennzeichnet, daß** das Verriegelungsteil (17, 17') in einer der Oberseite (19) benachbarten Führung (24) verschiebbar gelagert ist.

12. Kniegelenkendoprothese nach Anspruch 11, **dadurch gekennzeichnet, daß** das Verriegelungsteil (17, 17') in Richtung einer quer zur Achse (13, 13') angeordneten Beugeebene verschiebbar ist.

13. Kniegelenkendoprothese nach Anspruch 12, **dadurch gekennzeichnet, daß** das Verriegelungsteil (17) auf seiner dem Tibiateil (3) zugewandten Unterseite (25) eine auf die Achse (13) abgestimmte Ausformung (26) aufweist, die die Achse (13) in Richtung ihrer Lagerstelle (27) im Lagerraum (10) beaufschlagt.

14. Kniegelenkendoprothese nach Anspruch 12 oder 13, **dadurch gekennzeichnet, daß** das Verriegelungsteil (17) in einem Abstand zu der Ausformung (26) eine etwa parallel zur Achse (13) angeordnete Längsnut (28) aufweist, in die ein exzentrisch gelagerter Nocken (23) eines Exzenters (30) eingreift.

15. Kniegelenkendoprothese nach Anspruch 14, **dadurch gekennzeichnet, daß** der Nocken (29) exzentrisch auf einer der Oberseite (19) zugewandten oberen Seite (32) einer zylindrischen Scheibe (31) angeordnet ist, die auf einer der oberen Seite (32) abgewandten unteren Seite (33) eine Aufnahme (34) für ein Werkzeug aufweist.

16. Kniegelenkendoprothese nach Anspruch 14 oder 15, **dadurch gekennzeichnet, daß** der Exzenter (30) an einem oberen Ende eines von einer der Oberseite (19) des Gehäuses (2) abgewandten Unterseite (23) des Gehäuses (2) in Richtung Oberseite (19) ausgehenden Kanales (36) angeordnet ist.

17. Kniegelenkendoprothese nach Anspruch 15 oder 16, **dadurch gekennzeichnet, daß** das Verriegelungsteil (17) durch Vor schwenken des Exzenters (30) mit einem in den Kanal (36) einführbaren Werkzeug verschiebbar ist.

18. Kniegelenkendoprothese nach einem der Ansprüche 1 bis 17, **dadurch gekennzeichnet, daß** die Achse (13) an ihren einander abgewandten Enden Führungsflächen (15) aufweist, die im wesentlichen kugelabschnittsförmig ausgebildet sind.

19. Kniegelenkendoprothese nach einem der Ansprüche 1 bis 17, **dadurch gekennzeichnet, daß** die Achse (13') an ihren einander abgewandten Enden Führungsflächen (15') aufweist, die als Kreisringabschnitte (89) ausgebildet sind.

20. Kniegelenkendoprothese nach einem der Ansprüche 1 bis 19, **dadurch gekennzeichnet, daß** das Verriegelungsteil (17, 17') in dem Gehäuse (2, 2') einrastbar ist.

21. Kniegelenkendoprothese nach Anspruch 19 oder 20, **dadurch gekennzeichnet, daß** zum Einzementieren des Femurteiles (1) das Gehäuse (2') durch einen provisorischen Deckel (90)abdeckbar ist.

22. Kniegelenkendoprothese nach Anspruch 21, **dadurch gekennzeichnet, daß** der provisorische Deckel (90) in die Führung (24) des Gehäuses (2') einsetzbar ist.

23. Kniegelenkendoprothese nach Anspruch 1 bis 22, **dadurch gekennzeichnet, daß** das Femurgleitlager (37) ventral in einen kondylären Bereich übergeht, der als Patellaschild (48) ausgebildet ist.

24. Kniegelenkendoprothese nach Anspruch 23, **dadurch gekennzeichnet, daß** das Femurgleitlager (37) zwei in einem Abstand zueinander verlaufende innere Auflageflächen (49) aufweist.

25. Kniegelenkendoprothese nach einem der Ansprüche 1 bis 24, **dadurch gekennzeichnet, daß** das Femurgleitlager (37) als zwei Flügel (40) ausgebildet ist, die auf ihrer der Auflagefläche (41) abgewandten Innenfläche (50) als äußere Begrenzung eine von der Innenfläche (50) weggerichtete Randlippe (51) aufweisen.

26. Kniegelenkendoprothese nach Anspruch 25, **dadurch gekennzeichnet; daß** die Innenfläche (50) eine Vielzahl von Ausformungen zur besseren Verbindung mit dem an ihr anliegenden Material aufweist.

27. Kniegelenkendoprothese nach Anspruch 26, **dadurch gekennzeichnet, daß** die Ausformungen als gewellte Schneidkanten (100) ausgebildet sind.

28. Kniegelenkendoprothese nach einem der Ansprüche 1 bis 27, **dadurch gekennzeichnet, daß** das Tibiateil (3) auf einer einer Tibia (72) zugewandten Unterfläche (101) mit einer von der Unterfläche (101) abgewandten Randlippe (51) zur äußeren Begrenzung versehen ist.

29. Kniegelenkendoprothese nach Anspruch 28, **dadurch gekennzeichnet, daß** auf der Unterfläche (101) eine Vielzahl von Ausformungen zur formschlüssigen Verbindung mit dem an ihr anliegenden Material vorgesehen sind.

30. Kniegelenkendoprothese nach Anspruch 29, **dadurch gekennzeichnet, daß** die Ausformungen als gewellte Schneidkanten (100) ausgebildet sind.

31. Kniegelenkendoprothese nach einem der Ansprüche 1 bis 30, **dadurch gekennzeichnet, daß** das Tibiaplateau (38) eine Tibiaauflagefläche (52) aufweist, die auf das Femurgleitlager (37) abgestimmt ist.

32. Kniegelenkendoprothese nach Anspruch 31, **dadurch gekennzeichnet, daß** die Tibiaauflagefläche (52) eine auf die Eindellung (47) der Auflagefläche (41) des Femurgleitlagers (37) abgestimmte Erhebung (53) aufweist, gegen die die Eindellung (47) anschlagbar ist, und die gemeinsam mit der Eindellung (47) eine Vergrößerung der auf dem Tibiaplateau (38) ausgebildeten Auflagefläche darstellt.

33. Kniegelenkendoprothese nach einem der Ansprüche 1 bis 32, **dadurch gekennzeichnet, daß** dem Femurgleitlager (37) ventral in einem der Patella (20) zugewandten kondilären Bereich eine Patellainterpositionsprothese (54) vorgelagert ist.

34. Kniegelenkendoprothese nach Anspruch 33, **dadurch gekennzeichnet, daß** die Patellainterpositionsprothese (54) in einem ventralen Bereich des Tibiaplateaus (38) angelenkt ist.

35. Kniegelenkendoprothese nach Anspruch 33 oder 34, **dadurch gekennzeichnet, daß** die Patellainterpositionsprothese (54) um mindestens eine Schwenkachse (56) schwenkbar ist, die etwa parallel zu einer Kniegelenkschwenkachse (6) verläuft.

36. Kniegelenkendoprothese nach Anspruch 33 oder 35, **dadurch gekennzeichnet, daß** die Patellainterpositionsprothese (54) über mindestens ein Zwischenstück (55) mit dem Tibiaplateau (38) verbunden ist.

37. Kniegelenkendoprothese nach einem der Ansprüche 33 bis 36, **dadurch gekennzeichnet, daß** die Patellainterpositionsprothese (54) mit einer der Patella (20) abgewandten Rückfläche (59) auf dem Patellaschild (48) gleitbar anliegt.

38. Kniegelenkendoprothese nach einem der Ansprüche 33 bis 37, **dadurch gekennzeichnet, daß** die Patellainterpositionsprothese (54) auf ihrer der Rückfläche (59) abgewandten Seite eine Auflagefläche (60) aufweist, auf die die Patella (20) mit ihrer der Patellainterpositionsprothese (54) zugewand ten Patellarückfläche (61) großflächig lagerbar ist.

39. Kniegelenkendoprothese nach einem der Ansprüche 1 bis 38, **dadurch gekennzeichnet, daß** das Femurteil (1) an seinem dem Tibiateil (3) abgewandten Ende einen auswechselbaren Femurschaft (7) aufweist.

40. Kniegelenkendoprothese nach Anspruch 39, **dadurch gekennzeichnet, daß** der Femurschaft (7) mit seinem gelenkseitigen Ende (62) auf einen fest mit dem Femurteil (1) verbundenen Kegel (63) aufsteckbar ist.

41. Kniegelenkendoprothese nach einem der Ansprüche 1 bis 40, **dadurch gekennzeichnet, daß** das Tibiateil (3) an seinem dem Femurteil (1) abgewandten Ende einen auswechselbaren Tibiaschaft (8) aufweist.

42. Kniegelenkendoprothese nach Anspruch 41, **dadurch gekennzeichnet, daß** der Tibiaschaft (8) mit seinem gelenkseitigen Ende (64) auf einen fest mit dem Tibiateil (8) verbundenen Kegel (65) aufsteckbar ist.

43. Kniegelenkendoprothese nach einem der Ansprüche 40 bis 42, **dadurch gekennzeichnet, daß** die Kegel (63, 65) als stumpfe Kegel ausgebildet sind.

44. Kniegelenkendoprothese nach einem der Ansprüche 40 bis 43, **dadurch gekennzeichnet, daß** die Schäfte (7, 8) eine Längsbohrung (66) aufweisen, durch die eine Schraube (67) einführbar ist, mit der die Schäfte (7, 8) mit dem Kegel (63, 65) verschraubbar sind.

45. Kniegelenkendoprothese nach Anspruch 44, **dadurch gekennzeichnet, daß** die Schrauben (67) in ihrer Längsrichtung eine Entlüftungsbohrung (68) aufweisen.

46. Kniegelenkendoprothese nach einem der Ansprüche 1 bis 45, **dadurch gekennzeichnet, daß** das Femurteil (1) mit seinem Femurschaft (7) in einen vorbereiteten Knochenkanal (69) des benachbarten Femurs (70) einzementierbar ist.

47. Kniegelenkendoprothese nach Anspruch 46, **dadurch gekennzeichnet, daß** dem Femurschaft (7) ein in den Knochenkanal (69) einsetzbares Führungssystem (73) vorlagerbar ist.

48. Kniegelenkendoprothese nach einem der Ansprüche 1 bis 47, **dadurch gekennzeichnet, daß** das Tibiateil (3) mit seinem Tibiaschaft (8) in einen vorbereiteten Knochenkanal (71) der benachbarten Tibia (72) einzementierbar ist.

49. Kniegelenkendoprothese nach Anspruch 48, **dadurch gekennzeichnet, daß** dem Tibiaschaft (8) ein in den Knochenkanal (71) einsetzbares Führungssystem (73) vorlagerbar ist.

50. Kniegelenkendoprothese nach einem der Ansprüche 47 bis 49, **dadurch gekennzeichnet, daß** das Führungssystem (73) eine von einer zentralen Bohrung (74) durchzogene Führungsspindel (75) aufweist, an deren dem Schaft (8) zugewandten Ende (76) ein den Knochenkanal (69, 71) gegen Knochenzement (94) abdichtender Teller (77) angeordnet ist.

51. Kniegelenkendoprothese nach Anspruch 50, **dadurch gekennzeichnet, daß** die Führungsspindel (75) an ihrem dem Teller (77) abgewandten Ende (78) ein sich am Knochenkanal (69, 71) abstützendes Rossettensystem (80) aufweist.

52. Kniegelenkendoprothese nach Anspruch 51, **dadurch gekennzeichnet, daß** das Rossettensystem (80) quer zur Längsachse (79) der Führungsspindel (75) sternförmig ausgebildet ist.

53. Kniegelenkendoprothese nach Anspruch 52, **dadurch gekennzeichnet, daß** das Rossettensystem (80) vier um etwa 90° zueinander versetzte Auflageecken (88) aufweist.

54. Kniegelenkendoprothese nach einem der Ansprüche 50 bis 53, **dadurch gekennzeichnet, daß** die Führungsspindel (75) mit einem Einführinstrument (82) in den Knochenkanal (69, 71) einführbar ist.

55. Kniegelenkendoprothese nach Anspruch 54, **dadurch gekennzeichnet, daß** die Führungsspindel (75) auf ein vorderes Ende des Einführungsinstrumentes (82) aufschraubbar ist.

56. Kniegelenkendoprothese nach Anspruch 54 oder 55, **dadurch gekennzeichnet, daß** das Einführinstrument (82) einen Anschlag (84) aufweist, der auf die Tiefe des in den Knochenkanal (69, 71) einzuführenden Schaftes (7, 8) einstellbar ist.

57. Kniegelenkendoprothese nach einem der Ansprüche 54 bis 56, **dadurch gekennzeichnet, daß** nach dem Entfernen des Einführingtrumentes (82) ein Führungsstab (85) mit einem vorderen Ende (86) in die zentrale Bohrung (74) der Führungspindel (75) einsetzbar ist.

58. Kniegelenkendoprothese nach Anspruch 57, **dadurch gekennzeichnet, daß** über ein dem vorderen Ende (86) benachbartes hinteres Ende (87) des Führungsstabes (85) der Schaft (7, 8) in den Knochenkanal (69, 71) einsetzbar ist.

## Claims

1. Knee endoprosthesis comprising a femur part (1) with housing (2) and a tibia part (3) with a shaft (13) secured to a support (4), the shaft being mounted so as to be swivellable in the housing 2 in a bending plane (5), the femur part (1) being mounted with a femur sliding bearing (37) so as to slide on a tibia plateau connected to the tibia part (3) and at its dorsal end (43) has a steeper curvature than at its ventral end (44) and at the transition from the steeper to the flatter curvature there is provided an indentation (47) into which, in a stretched position, an elevation (53) of the tibia plateau (38) projects, **characterised in that** the support (4), with a shaft (13) permanently arranged on the support, can be inserted into an access channel (11, 21) of the housing (2) and can be locked in the housing (2) and the femur sliding bearing (37) comprises at least one inner bearing surface (49) that runs parallel to the bending plane (5) and in the region of which the flatter curvature is displaced into the region of the steeper curvature.

2. Knee endoprosthesis according to claim 1, **characterised in that** the housing (2, 2') comprises a bearing space into which the shaft (13) may be inserted so as to be engaged.

3. Knee endoprosthesis according to either claim 1 or 2, **characterised in that** the support (4, 4') can be locked in the housing (2, 2', 2").

4. Knee endoprosthesis according to any one of claims 1 to 3, **characterised in that** the access channel (11, 21) may be at least partially covered by a cover (16, 16').

5. Knee endoprosthesis according to claim 4, **characterised in that** the cover (16, 16') is constructed as a locking part.

6. Knee endoprosthesis according to any one of claims 1 to 5, **characterised in that** the access channel (11') issues from an access (22) at a lower side (23) of the housing (2") that faces the tibia part (3).

7. Knee endoprosthesis according to claim 6, **characterised in that** the access (22) is arranged in a leading region of the lower side (23) of the housing (2") that faces the patella (20).

8. Knee endoprosthesis according to any one of claims 5 to 7, **characterised in that** the locking part (17, 17') can be inserted into the access channel (11, 21).

9. Knee endoprosthesis according to any one of claims 1 to 8, **characterised in that** the access channel (11) issues from an access (18) at a top side (19) of the housing (2, 2') that faces away from the tibia part (3).

10. Knee endoprosthesis according to claim 9, **characterised in that** the access (18) is arranged in a trailing region of the top side (19) of the housing (2, 2') that faces away from the patella (20).

11. Knee endoprosthesis according to claim 9 or 10, **characterised in that** locking part (17, 17') is displaceably mounted in a guide (24) adjacent to the top side (19).

12. Knee endoprosthesis according to claim 11, **characterised in that** the locking part (17, 17') is displaceable in a direction of a bending plane arranged transversely to the shaft (13, 13').

13. Knee endoprosthesis according to claim 12, **characterised in that** the locking part (17), on its lower side (25) facing the tibia part (3), comprises a moulding (26) matched to the shaft (13) and which loads the shaft (13) in the direction of its bearing (27) in the bearing space (10).

14. Knee endoprosthesis according to claim 12 or 13, **characterised in that** at a spacing from the moulding (26) the locking part (17) comprises a longitudinal groove (28) arranged approximately parallel to the shaft (13) and into which an eccentrically mounted cam (23) of an eccentric (30) engages.

15. Knee endoprosthesis according to claim 14, **characterised in that** the cam (29) is eccentrically arranged on an upper side (32) of a cylindrical disc (31) that faces the top side (19), the disc, on a lower side (33) that faces away from the upper side (32), comprising a receiver (34) for a tool.

16. Knee endoprosthesis according to claim 14 or 15, **characterised in that** the eccentric (30) is arranged at an upper end of a channel (36) issuing from a lower side (23) of the housing (2) that faces away from the top side (19) of the housing (2), in the direction of the top side (19).

17. Knee endoprosthesis according to claim 15 or 16, **characterised in that** the locking part (17) can be displaced by swivelling the eccentric (30) using a tool that may be introduced into the channel (36).

18. Knee endoprosthesis according to any one of claims 1 to 17, **characterised in that**, at its ends that face away from each other, the shaft (13) comprises guide faces (15) which are formed so as to be substantially in the shape of a spherical segment.

19. Knee endoprosthesis according to any one of claims 1 to 17, **characterised in that**, at its ends that face away from each other, the shaft (13') comprises guide faces (15') which are formed as ring sections (89).

20. Knee endoprosthesis according to any one of claims 1 to 19, **characterised in that** the locking part (17, 17') can engage in the housing (2, 2').

21. Knee endoprosthesis according to claim 19 or 20, **characterised in that** for cementing-in the femur part (1), the housing (2') may be covered by a temporary cover (90).

22. Knee endoprosthesis according to claim 21, **characterised in that** the temporary cover (90) can be inserted into the guide (24) of the housing (2').

23. Knee endoprosthesis according to claim 1 to 22, **characterised in that** the femur sliding bearing (37) merges ventrally into a condylar region which is constructed as a patella shield (48).

24. Knee endoprosthesis according to claim 23, **characterised in that** the femur sliding bearing (37) comprises two inner bearing surfaces (49) that extend at a spacing from each other.

25. Knee endoprosthesis according to any one of claims 1 to 24, **characterised in that** the femur sliding bearing (37) is constructed as two wings (40) which on their inner face (50) facing away from the bearing surface (41) comprise, as an outer delimitation, an edge lip (51), which points away from the inner face (50).

26. Knee endoprosthesis according to claim 25, **characterised in that** the inner face (50) comprises a large number of mouldings for improved connection with the material resting thereon.

27. Knee endoprosthesis according to claim 26, **characterised in that** the mouldings are constructed as undulating cutting edges (100).

28. Knee endoprosthesis according to any one of claims 1 to 27, **characterised in that**, on a lower face (101) that faces a tibia (72), the tibia part (3) is provided with an edge lip (51), which faces away from the lower face (101), for outer delimitation.

29. Knee endoprosthesis according to claim 28, **characterised in that** a large number of mouldings are provided on the lower face (101) for interlocking connection with the material resting thereon.

30. Knee endoprosthesis according to claim 29, **characterised in that** the mouldings are constructed as undulating cutting edges (100).

31. Knee endoprosthesis according to any one of claims 1 to 30, **characterised in that** the tibia plateau (38) comprises a tibia bearing surface (52) which is matched to the femur sliding bearing (37).

32. Knee endoprosthesis according to claim 31, **characterised in that** the tibia bearing surface (52) comprises an elevation (53) matched to the indentation (47) of the bearing surface (41) of the femur sliding bearing (37) and against which the indentation (47) can abut, and which together with the indentation (47) constitutes an enlargement of the bearing surface formed on the tibia plateau (38).

33. Knee endoprosthesis according to any one of claims 1 to 32, **characterised in that** a patella interposition prosthesis (54) precedes the femur sliding bearing (37) ventrally in a condylar region that faces the patella (20).

34. Knee endoprosthesis according to claim 33, **characterised in that** the patella interposition prosthesis (54) is articulated in a ventral region of the tibia plateau (38).

35. Knee endoprosthesis according to claim 33 or 34, **characterised in that** the patella interposition prosthesis (54) can be swivelled about at least one swivelling axis (56) that runs approximately parallel to a knee joint swivelling axis (6).

36. Knee endoprosthesis according to claim 33 or 35, **characterised in that** the patella interposition prosthesis (54) is connected to the tibia plateau (38) by at least one spacer (55).

37. Knee endoprosthesis according to any one of claims 33 to 36, **characterised in that**, with a back face (59) that faces away from the patella (20), the patella interposition prosthesis (54) slidably rests on the patella shield (48).

38. Knee endoprosthesis according to any one of claims 33 to 37, **characterised in that**, on its side facing away from the back face (59), the patella interposition prosthesis (54) comprises a bearing surface (60) onto which the patella (20), with its patella back face (61) that faces the patella interposition prosthesis (54), can be superposed over a large area.

39. Knee endoprosthesis according to any one of claims 1 to 38, **characterised in that**, at its end that faces the tibia part (3), the femur part (1) comprises a replaceable femur shank (7).

40. Knee endoprosthesis according to claim 39, **characterised in that** with its joint-side end (62) the femur shank (7) can be placed onto a cone (63) that is permanently connected to the femur part (1).

41. Knee endoprosthesis according to any one of claims 1 to 40, **characterised in that**, at its end that faces away from the femur part (1), the tibia part (3) comprises a replaceable tibia shank (8).

42. Knee endoprosthesis according to claim 41, **characterised in that** with its joint-side end (64) the tibia shank (8) can be placed onto a cone (65) that is permanently connected to the tibia part (8).

43. Knee endoprosthesis according to any one of claims 40 to 42, **characterised in that** the cone (63, 65) is constructed as a truncated cone.

44. Knee endoprosthesis according to any one of claims 40 to 43, **characterised in that** the shanks (7, 8) comprise a longitudinal hole (66) through which a screw (67) can be introduced with which the shanks (7, 8) can be screwed to the cone (63, 65).

45. Knee endoprosthesis according to claim 44, **characterised in that** in their longitudinal direction the screws (67) comprise a ventilation hole (68).

46. Knee endoprosthesis according to any one of claims 1 to 45, **characterised in that** with its femur shank (7) the femur part (1) can be cemented into a prepared bone channel (69) of the adjacent femur (70).

47. Knee endoprosthesis according to claim 46, **characterised in that** a guide system (73) that can be inserted into the bone channel (69) can precede the femur shank (7).

48. Knee endoprosthesis according to any one of claims 1 to 47, **characterised in that** with its tibia shank (8) the tibia part (1) can be cemented into a prepared bone channel (71) of the adjacent tibia (72).

49. Knee endoprosthesis according to claim 48, **characterised in that** a guide system (73) that can be inserted into the bone channel (71) can precede the tibia shank (8).

50. Knee endoprosthesis according to any one of claims 47 to 49, **characterised in that** the guide system (73) comprises a guide spindle (75) penetrated by a central hole (74) and on the end (76) of which that faces the shank (8) is arranged a plate (77) that seals the bone channel (69, 71) from bone cement (94).

51. Knee endoprosthesis according to claim 50, **characterised in that**, at its end (78) that faces away from the plate (77), the guide spindle (75) comprises a rosette system (80) supported on the bone channel (69, 71).

52. Knee endoprosthesis according to claim 51, **characterised in that** the rosette system (80) is star-shaped transversely to the longitudinal axis (79) of the guide spindle (75).

53. Knee endoprosthesis according claim 52, **characterised in that** the rosette system (80) comprises four bearing corners (88) that are mutually offset by approximately 90°.

54. Knee endoprosthesis according to any one of claims 50 to 53, **characterised in that** the guide spindle (75) can be introduced into the bone channel (69, 71) using an introduction tool (82).

55. Knee endoprosthesis according to claim 54, **characterised in that** the guide spindle (75) can be screwed onto a leading end of the introduction tool (82).

56. Knee endoprosthesis according to claim 54 or 55, **characterised in that** the introduction tool (82) comprises a stop (84) which can be adjusted to the depth of the shank (7, 8) that is to be introduced into the bone channel (69, 71).

57. Knee endoprosthesis according to any one of claims 54 to 56, **characterised in that** after removing the introduction tool (82) a guide bar (85) can be inserted with a leading end (86) into the central hole (74) of the guide spindle (75).

58. Knee endoprosthesis according to claim 57, **characterised in that** the shank (7, 8) can be inserted into the bone channel (69, 71) via a trailing end (87) of the guide bar (85) that is adjacent to the leading end (86).

## Revendications

1. Endoprothèse de genou composée d'une partie fémorale (1) avec un logement (2) et d'une partie tibiale (3) avec un axe (13) fixé sur un support (4), qui est supporté dans le logement (2) de manière à pouvoir pivoter dans un plan de flexion (5), dans laquelle la partie fémorale (1) est supportée de façon glissante par un palier de glissement fémoral (37) sur un plateau tibial relié à la partie tibiale (3) et présente à son extrémité dorsale (43) une plus forte courbure qu'à son extrémité ventrale (44) et il est prévu à la transition entre la courbure forte et la courbure moins forte un creux (47) dans lequel une saillie (53) du plateau tibial (38) dépasse dans une position d'extension, **caractérisée en ce que** le support (4) peut être inséré avec un axe (13) disposé fixe sur le support dans un canal d'accès (11, 21) du logement (2) et verrouillé dans le logement (2) et le palier de glissement fémoral (37) possède au moins une surface d'appui intérieure (49) parallèle au plan de flexion (5), au niveau de laquelle la courbure moins forte est décalée dans la zone de la courbure plus forte.

2. Endoprothèse de genou selon la revendication 1, **caractérisée en ce que** le logement (2, 2') possède un compartiment d'appui dans lequel l'axe (13) peut être introduit et emboîté.

3. Endoprothèse de genou selon l'une des revendications 1 ou 2, **caractérisée en ce que** le support (4, 4') peut être verrouillé dans le logement (2, 2', 2").

4. Endoprothèse de genou selon l'une des revendications 1 à 3, **caractérisée en ce que** le canal d'accès (11, 21) peut être recouvert au moins partiellement par une couverture (16, 16').

5. Endoprothèse de genou selon la revendication 4, **caractérisée en ce que** la couverture (16, 16') est conformée comme une partie de verrouillage.

6. Endoprothèse de genou selon l'une des revendications 1 à 5, **caractérisée en ce que** le canal d'accès (11") part d'un accès (22) sur une face inférieure (23) du logement (2") orientée vers la partie tibiale (3).

7. Endoprothèse de genou selon la revendication 6, **caractérisée en ce que** l'accès (22) est disposé dans une zone antérieure, tournée vers la rotule (20), de la face inférieure (23) du logement (2").

8. Endoprothèse de genou selon l'une des revendications 5 à 7, **caractérisée en ce que** la partie de verrouillage (17, 17') peut être introduite dans le canal d'accès (11, 21).

9. Endoprothèse de genou selon l'une des revendications 1 à 8, **caractérisée en ce que** le canal d'accès (11) part d'un accès (18) sur une face supérieure (19) du logement (2, 2') orientée à l'opposé de la partie tibiale (3).

10. Endoprothèse de genou selon la revendication 9, **caractérisée en ce que** l'accès (18) est disposé dans une partie postérieure, orientée à l'opposé de la rotule (20), de la face supérieure (19) du logement (2, 2').

11. Endoprothèse de genou selon la revendication 9 ou 10, **caractérisée en ce que** la partie de verrouillage (17, 17') est supportée avec possibilité de translation dans un guide (24) voisin de la face supérieure (19).

12. Endoprothèse de genou selon la revendication 11, **caractérisée en ce que** la partie de verrouillage (17, 17') peut se déplacer dans la direction d'un plan de flexion disposé perpendiculairement à l'axe (13, 13').

13. Endoprothèse de genou selon la revendication 12, **caractérisée en ce que** la partie de verrouillage (17) présente sur sa face inférieure (25) orientée vers la partie tibiale (3) un relief (26) adapté à l'axe (13), qui agit sur l'axe (13) en direction de son point d'appui (27) dans le compartiment d'appui (10).

14. Endoprothèse de genou selon la revendication 12 ou 13, **caractérisée en ce que** la partie de verrouillage (17) possède à distance du relief (26) une gorge allongée (28) approximativement parallèle à l'axe (13), qui se met en prise avec une came (23) excentrée d'un excentrique (30).

15. Endoprothèse de genou selon la revendication 14, **caractérisée en ce que** la came (29) est disposée en position excentrée sur une face supérieure (32), tournée vers la face supérieure (19), d'un disque cylindrique (31) qui possède sur une face inférieure (33) orientée à l'opposé de la face supérieure (32) un réceptacle (34) pour un outil.

16. Endoprothèse de genou selon la revendication 14 ou 15, **caractérisée en ce que** l'excentrique (30) est disposé à une extrémité supérieure d'un canal (36) partant de la face inférieure (23) du logement (2) opposée à la face supérieure (19) du logement (2) en direction de la face supérieure (19).

17. Endoprothèse de genou selon la revendication 15 ou 16, **caractérisée en ce que** la partie de verrouillage (17) peut être déplacée en faisant basculer l'excentrique (30) vers l'avant avec un outil pouvant être introduit dans le canal (36).

18. Endoprothèse de genou selon l'une des revendications 1 à 17, **caractérisée en ce que** l'axe (13) possède à ses extrémités opposées l'une à l'autre des surfaces de guidage (15) qui sont pour l'essentiel en forme de segments de sphère.

19. Endoprothèse de genou selon l'une des revendications 1 à 17, **caractérisée en ce que** l'axe (13') possède à ses extrémités opposées l'une à l'autre des surfaces de guidage (15') conformées comme des segments d'anneau circulaire (89).

20. Endoprothèse de genou selon l'une des revendications 1 à 19, **caractérisée en ce que** la partie de verrouillage (17, 17') peut être emboîtée dans le logement (2, 2').

21. Endoprothèse de genou selon la revendication 19 ou 20, **caractérisée en ce qu'**en vue du scellement de la partie fémorale (1), le logement (2') peut être couvert par un couvercle provisoire (90).

22. Endoprothèse de genou selon la revendication 21, **caractérisée en ce que** le couvercle provisoire (90) peut être inséré dans le guide (24) du logement (2').

23. Endoprothèse de genou selon la revendication 1 à 22, **caractérisée en ce que** le palier de glissement fémoral (37) est suivi dans le sens ventral par une zone condylaire qui est conformée comme un bouclier rotulien (48).

24. Endoprothèse de genou selon la revendication 23, **caractérisée en ce que** le palier de glissement fémoral (37) possède deux surfaces d'appui intérieures (49) situées à distance l'une de l'autre.

25. Endoprothèse de genou selon l'une des revendications 1 à 24, **caractérisée en ce que** le palier de glissement fémoral (37) est conformé comme deux ailes (40) qui possèdent sur leur surface intérieure (50) opposée à la surface d'appui (41) pour servir de délimitation extérieure une lèvre marginale (51) orientée à l'opposé de la surface intérieure (50).

26. Endoprothèse de genou selon la revendication 25, **caractérisée en ce que** la surface intérieure (50) présente une pluralité de reliefs pour une meilleure liaison avec le matériau adjacent.

27. Endoprothèse de genou selon la revendication 26, **caractérisée en ce que** les reliefs sont conformés comme des bords coupants (100) ondulés.

28. Endoprothèse de genou selon l'une des revendications 1 à 27, **caractérisée en ce que** la partie tibiale (3) est dotée sur une face inférieure (101) orientée vers le tibia (72) d'une lèvre marginale (51) dirigée à l'opposé de la face inférieure (101) pour la délimitation vers l'extérieur.

29. Endoprothèse de genou selon la revendication 28, **caractérisée en ce qu'**il est prévu sur la surface inférieure (101) une pluralité de reliefs pour l'assemblage par engagement positif avec le matériau adjacent.

30. Endoprothèse de genou selon la revendication 29, **caractérisée en ce que** les reliefs sont conformés comme des bords coupants (100) ondulés.

31. Endoprothèse de genou selon l'une des revendications 1 à 30, **caractérisée en ce que** le plateau tibial (38) possède une surface d'appui tibial (52) qui est adaptée au palier de glissement fémoral (37).

32. Endoprothèse de genou selon la revendication 31, **caractérisée en ce que** la surface d'appui tibial (52) possède une saillie (53) adaptée au creux (47) de la surface d'appui (41) du palier de glissement fémoral (37) contre laquelle le creux (47) peut buter, et qui constitue avec le creux (47) une augmentation de la surface d'appui formée sur le plateau tibial (38).

33. Endoprothèse de genou selon l'une des revendications 1 à 32, **caractérisée en ce qu'**une prothèse intercalaire rotulienne (54) est disposée devant le palier de glissement fémoral (37) au niveau ventral dans une zone condylaire orientée vers la rotule (20).

34. Endoprothèse de genou selon la revendication 33, **caractérisée en ce que** la prothèse intercalaire rotulienne (54) est articulée dans une partie ventrale du plateau tibial (38).

35. Endoprothèse de genou selon la revendication 33 ou 34, **caractérisée en ce que** la prothèse intercalaire rotulienne (54) peut pivoter au moins autour d'un axe de pivotement (56) qui est approximativement parallèle à l'axe de pivotement (6) de l'articulation du genou.

36. Endoprothèse de genou selon la revendication 33 ou 35, **caractérisée en ce que** la prothèse intercalaire rotulienne (54) est reliée par au moins un élément intercalaire (55) au plateau tibial (38).

37. Endoprothèse de genou selon l'une des revendications 33 à 36, **caractérisée en ce que** la prothèse intercalaire rotulienne (54) repose de façon coulissante sur le bouclier rotulien (48) par une face arrière (59) orientée à l'opposé de la rotule (20).

38. Endoprothèse de genou selon l'une des revendications 33 à 37, **caractérisée en ce que** la prothèse intercalaire rotulienne (54) possède sur sa face opposée à la face arrière (59) une surface d'appui (60) sur laquelle la rotule (20) repose sur une large surface par sa face postérieure (61) tournée vers la prothèse intercalaire rotulienne (54).

39. Endoprothèse de genou selon l'une des revendications 1 à 38, **caractérisée en ce que** la partie fémorale (1) possède à son extrémité opposée à la partie tibiale (3) une tige fémorale (7) interchangeable.

40. Endoprothèse de genou selon la revendication 39, **caractérisée en ce que** la tige fémorale (7) peut être emboîtée à son extrémité articulaire (62) sur un cône (63) solidaire de la partie fémorale (1).

41. Endoprothèse de genou selon l'une des revendications 1 à 40, **caractérisée en ce que** la partie tibiale (3) présente à son extrémité opposée à la partie fémorale (1) une tige tibiale (8) interchangeable.

42. Endoprothèse de genou selon la revendication 41, **caractérisée en ce que** la tige tibiale (8) peut être emboîtée à son extrémité articulaire (64) sur un cône (65) fixé à demeure sur la partie tibiale (8).

43. Endoprothèse de genou selon l'une des revendications 40 à 42, **caractérisée en ce que** les cônes (63, 65) sont conformés comme des troncs de cône.

44. Endoprothèse de genou selon l'une des revendications 40 à 43, **caractérisée en ce que** les tiges (7, 8) possèdent un alésage longitudinal (66) à travers lequel peut être introduite une vis (67) servant à visser les tiges (7, 8) sur les cônes (63, 65).

45. Endoprothèse de genou selon la revendication 44, **caractérisée en ce que** les vis (67) possèdent dans leur sens longitudinal un alésage de sortie d'air (68).

46. Endoprothèse de genou selon l'une des revendications 1 à 45, **caractérisée en ce que** la partie fémorale (1) peut être scellée avec sa tige fémorale (7) dans un canal osseux (69) préparé dans le fémur (70) voisin.

47. Endoprothèse de genou selon la revendication 46, **caractérisée en ce qu'**un système de guidage (73) pouvant être introduit dans le canal osseux (69) peut être placé avant la tige fémorale (7).

48. Endoprothèse de genou selon l'une des revendications 1 à 47, **caractérisée en ce que** la partie tibiale (3) peut être scellée avec sa tige tibiale (8) dans un canal osseux (71) préparé dans le tibia (72) voisin.

49. Endoprothèse de genou selon la revendication 48, **caractérisée en ce qu'**un système de guidage (73) pouvant être introduit dans le canal osseux (71) peut être placé avant la tige tibiale (8).

50. Endoprothèse de genou selon l'une des revendications 47 à 49, **caractérisée en ce que** le système de guidage (73) comprend une tige de guidage (74) parcourue par un alésage central (75), dont l'extrémité (76) dirigée vers la tige (8) porte un plateau (77) qui assure l'étanchéité du canal osseux (69, 71) au ciment osseux (94).

51. Endoprothèse de genou selon la revendication 50, **caractérisée en ce que** la tige de guidage (75) présente à son extrémité (78) opposée au plateau (77) un système en rosette (80) qui s'appuie sur le canal osseux (69, 71).

52. Endoprothèse de genou selon la revendication 51, **caractérisée en ce que** le système en rosette (80) est en forme d'étoile perpendiculairement à l'axe longitudinal (79) de la tige de guidage (75).

53. Endoprothèse de genou selon la revendication 52, **caractérisée en ce que** le système en rosette (80) présente quatre coins d'appui (88) décalés entre eux de 90° environ.

54. Endoprothèse de genou selon l'une des revendications 50 à 53, **caractérisée en ce que** la tige de guidage (75) peut être introduite à l'aide d'un instrument d'insertion (82) dans le canal osseux (69, 71).

55. Endoprothèse de genou selon la revendication 54, **caractérisée en ce que** la tige de guidage (75) peut être vissée sur une extrémité antérieure de l'instrument d'insertion (82).

56. Endoprothèse de genou selon la revendication 54 ou 55, **caractérisée en ce que** l'instrument d'introduction (82) possède une butée (84) qui est réglable à la profondeur de la tige (7, 8) à introduire dans le canal osseux (69, 71).

57. Endoprothèse de genou selon l'une des revendications 54 à 56, **caractérisée en ce qu'**après le retrait de l'instrument d'insertion (82), une barre de guidage (85) peut être introduite par une extrémité antérieure (86) dans l'alésage central (74) de la tige de guidage (75).

58. Endoprothèse de genou selon la revendication 57, **caractérisée en ce que** la tige (7, 8) peut être introduite dans le canal osseux (69, 71) par-dessus une extrémité arrière (87) de la barre de guidage (85) voisine de l'extrémité avant (86).
